# EUROPEAN PATENT APPLICATION

(11) **EP 2 251 058 A1**
(43) Date of publication of application: **17.11.2010**
(21) Application number: 08736669.6
(22) Date of filing: 08.02.2008
(51) Int. Cl.: A61M 25/08, A61B 18/22

(54) **IMPROVED TRACTION DEVICE APPLICABLE TO A CONDUCTOR CABLE FOR ENDOVENOUS TREATMENTS**

(71) Applicant: Arcusa Villacampa, Francisco Javier, 08029 Barcelona (ES); Franco Lissott, Mailin Auxiliadora, 08029 Barcelona (ES); Leal Monedero, Javier, 08029 Barcelona (ES)
(72) Inventor: Arcusa Villacampa, Francisco Javier, 08029 Barcelona (ES); Franco Lissott, Mailin Auxiliadora, 08029 Barcelona (ES); Leal Monedero, Javier, 08029 Barcelona (ES)
(74) Representative: Morgades y Manonelles, Juan Antonio
(86) International application number: PCT/ES2008/000065
(87) International publication number: WO 2009/098328

(57) **Abstract**

The invention refers to a device made up of some driving devices for a fibre optic conductor cable, with galvanic current, RF current, ultra-sound or any other type of appropriate energy for the obliteration of a vein, by applying energy that is absorbed by the vessel wall. The type of energy used can be light, electrical current, ultra-sound or any other type of energy that produces this retraction effect, the driving devices are controlled by electronic means which determine the amount of the advance or withdrawal as well stopping of said fibre optic cable on the inside of the probe. In parallel the fibre optic cable is connected to a generator of laser or RF energy or any other type of energy that is suitable for the case.

## Description

The invention refers to a device made up of some driving devices for a fibre optic conductor cable, for galvanic current, RF current, ultra-sound or any other type of energy for the obliteration of a vein, by applying energy that is absorbed by the vessel wall. The type of energy used can be light, electrical current, ultra-sound or any other type of energy that produces this retraction effect.

Said driving devices are controlled by some electronic means which determine the amount of the advance or withdrawal and stopping of said fibre optic cable on the inside of the probe. In parallel the fibre optic cable is connected to a generator of laser or RF energy or any other type of energy that is suitable for the case.

The purpose of the driving device is to provide a mechanism to push or to withdraw the cable that communicates electrically, optically, etc. with the laser or RF generator or other source of energy being used to close the vein, in such a way that it brings about a pulling of the fibre optic or the RF probe or any other type of element used to transmit energy from a generator to the vein, in this way the improved device allows the energy coming from the generator to be applied in a homogeneous, controlled and repeatable manner.

Thanks to the device of the present invention, the energy can be applied as follows:
- Step to step
- In a continuous manner
- According to a previously programmed pattern, to allow the energy to be varied as a function of the geometry of the vein (diameter, thickness of the wall, anatomical situation, etc.).

The device that is the object of the invention will replace the pulling force that has until the present been provided by the hands of the surgeon, with the added advantages that unlike this, the pulling speed will be possibly adjusted in a manual or automatic manner, for which the device will include a cable support, which will immobilise same and will have two sets of rollers with identical functions; both sets will work as roller guides, driving the fibre optic cable forwards or backwards, the cable being kept immobilised to the support by means of a fork-like member and the second set of rollers can currently work as a cable speed control and measure the speed of the advance or withdrawal of the cable, warning the surgeon in the event that in spite of the pushing action of the first rollers the cable is not advancing or withdrawing as a result of having becomed jammed inside the vein.

The rollers are facing each other and with their surfaces in contact with the track guide ribs, the inside of which guides a worm screw with the help of both respective bearings. In turn, said worm screw receives the push from an electro-motor by the driving action of a suitable rotating device.

When the worm screw rotates on the inside of the track guide it forces the support fitted with rollers to advance or withdraw, said fibre optic cable support being immobilised.

The pushing and regulation devices of the driving rollers will be made up by an electro-motor whose shaft is connected to a speed adjuster, and a circular distribution of Hall sensors that allow the number of rotations of the electro-motor, likewise its position, to be known, in addition to the rotating speed of the shaft of the speed adjuster, which in turn will be the rotation axle of the worm screw with the interposition between the worm screw and the track guide of respective bearings.

The rotating speed of the electro-motor shaft can be controlled, apart from the action of the speed adjuster, by a set of gears, or equally by a controller of the current intensity passing through the electro-motor.

The rotating speed control means for the shaft of the electro-motor include as well the previously stated possibility that the fibre optic cable may be advanced step by step, in a continuous manner or by means of a programmed pattern, in both directions of advance of said cable.

Other details and characteristics will be explained during the course of the description that is given below in which reference is made by way of being illustrative but without being by way of limitation of a preferred embodiment of the invention.

A detailed list of the main parts of the invention follows, said parts being shown in the following figures; (10) device, (11) driving support, (12) electro-motor, (13) flanges, (14) worm screw, (15) track guide, (16) ribs, (17) fork, (18) rollers, (19) speed reducer, (20) shaft, (21) arms, (22) arms, (23) openings, (24) nipple, (25) cavity, (26) rotation axle, (27) cross members, (28) cross members, (29) wings, (30) opening, (31) slot, (32) fibre optic cable, (33) cavity, (34) table, (35) patient, (36) leg, (37) incision, (38) table, (39) legs, (40) upper plane, (41) front part, (42) pins, (43) ends, (44) nuts, (45) Hall sensors.
Figure nº 1 is a front elevation view of the device (10), made from a track guide (15) along which the driving support (11) slides driven by the electro-motor (12).
Figure nº 2 is a front elevation view of the driving support (11), on the front part (41) of which there is the fork (17).
Figure Nº 3 is perspective view of the fork (17) in an open position.
Figure nº 4 is a side elevation diagrammatic view of the support (11) sliding on the track guide (15).
Figure nº 5 is a front diagrammatic elevation view of a table (38) on which the device (10) swings.
Figure nº 6 is a front view of a side elevation of a stretcher bed (34) on which there is a patient (35), and the device (10) installed on the table (38).

In one of the preferred embodiments of what is the object of this present invention, and as can be seen in figure nº 1, the device (10) is made up from a track guide (15) with its ribs (16) along which the driving support (11) slides, and in one of the ends of said track guide (15) there is some means so as to drive the driving support (11). Said driving means are made up of an electro-motor (12), whose shaft (20) is fitted to some means of transmission such as a worm screw (14), that rotates thanks to the action of said electro-motor (12), which, in turn, is fixed to some flanges (13), immobilised in a vertical position thanks to pins (42) whose threaded ends (43) are held to said flanges (13) by means of nuts(44).

The purpose of the means of driving the device (10) is to push a fibre optic cable held by the support (11), so as to transmit a lineal movement forwards or equally backwards to same, with the help of a fork (17) and a support (11) on which said fork is immobilised.

When the worm screw (14) rotates on the inside of the track guide (15) it forces the forward or backward movement of the support (11), inside of which there is a threaded drill hole (33) along which the worm screw (14) rotates, sliding along the driving support (11) with the help of horizontal ribs (16), see figure nº 4, helped by the rollers (18), it finally connects a set of them (18) to the device electronics (10) as a speed and direction circulation control for the support (11) along the track guide (15). As the support (11) advances or withdraws it forces the fibre optic cable (32) to go forwards or backwards with it, on being supported (32) to said support (11) with the help of the fork (17).

Usually the control of the worm screw (14) and its rotation are left to the electro-motor (12), the shaft (20) of which is kinetically connected to the worm screw (14) by means of the speed reducer (19) that acts as a speed changer of said worm screw (14), a circular distribution of Hall sensors (45) being incorporated into said electro-motor (12) and its shaft (20).

Said electronics is not shown in the figures, it sets the working time, stopping, pause, forward and backward movement of the cable (32) which are completely controllable by the surgeon.

A fork (17) has been provisioned in the support (11) for the purpose of holding the fibre optic cable (32), see figure nº 3, which is made up of two parts that are jointed around the rotation axle (26), some openings (23) having been placed in the stiffeners (21-22) which in combination with the slots (31) placed in the wings (29) also allow for the alignment of the fibre optic cable (32) without same being able to form loops.

The fork (17) is made up of some stiffeners (21-22) and some cross members (27-28) one part (45) folding up on the other (46), and (45) being immobilised to (46) with the help of the nipple (24) on entering into the cavity (25). In such a way that when one half (45) forms an angle of 0º with the other half (46), the fibre optic cable (32) remains on the inside of the openings (23) without the possibility of moving from the fork (17) and the support (11).

After an operation and before starting another, if it is required to change the fibre optic cable (32), the two halves (45) and (46) are separated and the cable (32) is released, likewise the slots (31) through the opening (30).

The purpose of the device (10) is as and how can be seen in figure nº 6, the introduction of the fibre optic cable (32) into the inside of a vein of the patient (35) through one of his/her legs (36) by means of an incision (37), when said patient (35) is laying on the stretcher (34). Said cable (32) is used once only and is supplied completely sterile therefore, in each operation it must be replaced for another one and installed in the support (11) and the fork (17).

In order to make the introduction of the fibre optic cable into the leg (36) of the patient (35) easier, there is the help of a table (38) on which the device (10) can pivot, the height of the legs (39) being able to be adjusted with some suitable means. As an alternative, the table (38) can be done away with, and a tripod used for the same purpose, this means keeping the horizontal plane of the device (10) with that of the leg of the patient (35) as and how shown in figure nº 6.

Having sufficiently described this present invention in combination with the attached figures it is easy to see that any modifications to the detail that are considered advisable can be introduced, provided that they do not alter the essence of the invention that is summarised in the following claims.

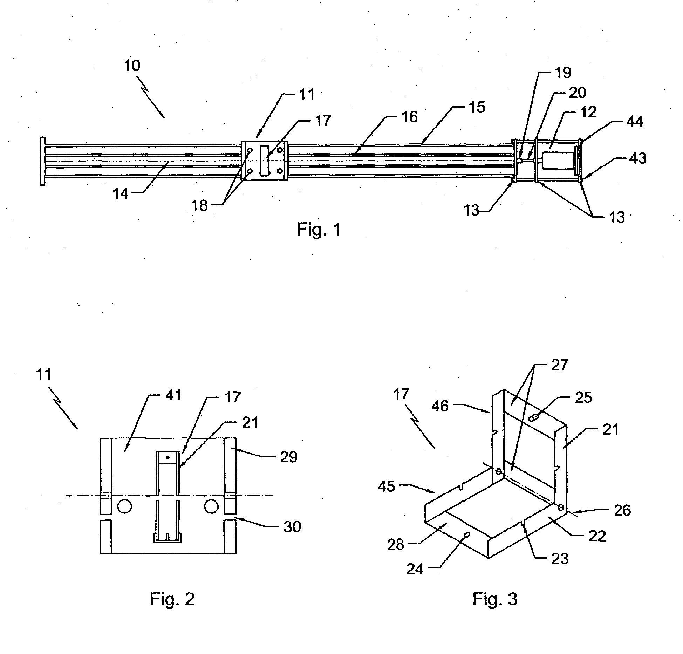

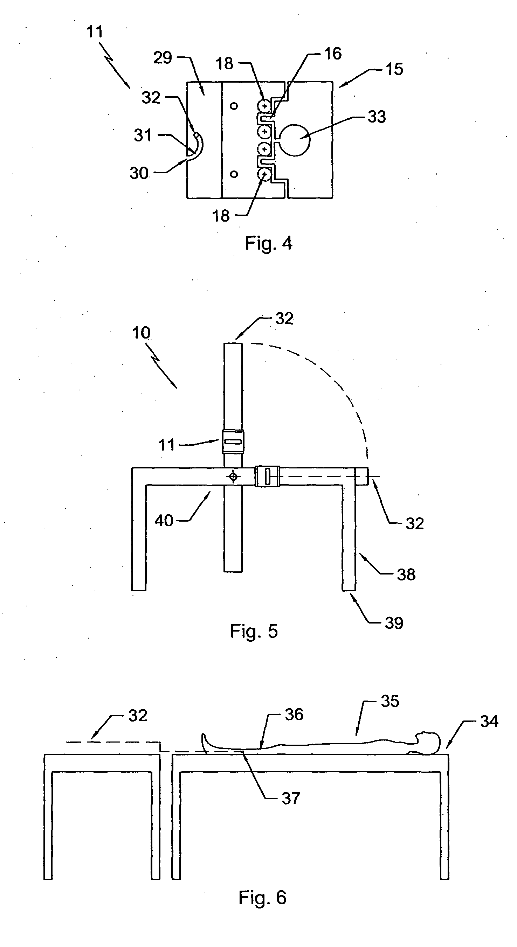

## Claims

1. "AN IMPROVED DRIVING DEVICE APPLICABLE TO A CONDUCTOR CABLE FOR INTRAVENOUS TREATMENTS" of the type using means of guiding a fibre optic cable to transmit energy to obliterate a vein, **characterised in that** said device (10) is made up of means for driving a fibre optic cable (32), means for immobilising said cable (32) to a sliding support (11), means for aligning the cable (32), and means for the programmed sliding of said support.

2. "AN IMPROVED DRIVING DEVICE APPLICABLE TO A CONDUCTOR CABLE FOR INTRAVENOUS TREATMENTS according to claim 1, **characterised in that** the fibre optic cable (32) will be of a type that is capable of conducting galvanic current, RF current and ultrasound.

3. "AN IMPROVED DRIVING DEVICE APPLICABLE TO A CONDUCTOR CABLE FOR INTRAVENOUS TREATMENTS" according to claim 1, **characterised in that** the energy supplied by the device (10) can be applied:
- Step by step.
- In a continuous manner.
- Following a previously programmed pattern.

4. "AN IMPROVED DRIVING DEVICE APPLICABLE TO A CONDUCTOR CABLE FOR INTRAVENOUS TREATMENTS" according to claim 1, **characterised in that** the means for driving the cable (32) in the device (10) is made up of an electro-motor (12) fitted to the device (10) by means of the vertical flanges (13) that are immobilised by means of pins (42) threaded at the ends (43) and secured by means of nuts (44).

5. "AN IMPROVED DRIVING DEVICE APPLICABLE TO A CONDUCTOR CABLE FOR INTRAVENOUS TREATMENTS" according to claim 1, **characterised in that** the means for immobilising the cable (32) to the support (11) are made up of a fork (17) formed from stiffeners (21-22) and cross members (27-28), one half (45) folding onto the other half (46) and being immobilised with the help of a nipple (24) on entering into the cavity (25), in such a way that when half (45) forms an angle of 0º with the other half (46), the fibre optic cable (32) remains on the inside of the openings (23) without the possibility of moving in respect of the fork (17).

6. " AN IMPROVED DRIVING DEVICE APPLICABLE TO A CONDUCTOR CABLE FOR INTRAVENOUS TREATMENTS" according to claims 1 and 5, **characterised in that** the fork (17) is made from two parts (45-46) that hinge on their rotation axle (26), some openings (23) having been provided in the stiffeners (21-22), which in combination with the groove (31) provided in the wings (29) also allow the fibre optic cable (32) to be aligned without same being able to form loops.

7. "AN IMPROVED DRIVING DEVICE APPLICABLE TO A CONDUCTOR CABLE FOR INTRAVENOUS TREATMENTS" according to claim 1, **characterised in that** the means for sliding the support (11) along the track guide (15) is made from a worm screw (14) which on rotating forces the forward or backward movement to the support (11), the inside of which has a threaded drill hole (33) through which it rotates (14), sliding (11) with the help of the horizontal ribs (16), helped by means of the rollers (18).

8. "AN IMPROVED DRIVING DEVICE APPLICABLE TO A CONDUCTOR CABLE FOR INTRAVENOUS TREATMENTS" according to claims 1 and 4, **characterised in that** the means of driving the device (10) are controlled by means of Hall sensors (45), distributed in a circular manner around the shaft (20) of the electro-motor (12), transferring the force of the shaft (20) to the worm screw (14) thanks to a reducer speed adjuster (19).
